# DEMANDE DE BREVET EUROPEEN

(11) **EP 1 933 218 A1**
(43) Date de publication de la demande: **18.06.2008**
(21) Numéro de dépôt: 07121608.9
(22) Date de dépôt: 27.11.2007
(51) Int. Cl.: G05D 11/13, G01N 33/00, G01N 33/98, G01N 33/497

(54) **Système et procédé de génération de mélange gazeux pour l'étalonnage d'éthylomètres portables**

(30) Priorité: 06.12.2006 FR 0655347
(71) Demandeur: Laboratoire National de Metrologie et d'Essais, 75015 Paris (FR)
(72) Inventeur: Couston, Alain, 92140 Clamart (FR)
(74) Mandataire: Casalonga, Axel

(57) **Abrégé**

Dispositif de production d'un mélange gazeux comprenant des régulateurs de débit massique gazeux pour réaliser l'admission de gaz entrant dans la composition du mélange, des régulateurs de débit massique liquide pour réaliser l'admission de liquides entrant dans la composition du mélange, un moyen (18) de contrôle de la température des gaz circulant dans le dispositif permettant d'éviter la condensation d'une partie du mélange gazeux et un moyen (19) de commande électronique permettant de régler et de contrôler en temps réel les paramètres de fonctionnement.

## Description

La présente invention concerne un système de génération de mélange gazeux pour l'étalonnage d'éthylomètres portables.

Les éthylomètres portables ont vu leur utilisation développée ces dernières années avec le durcissement de la législation en vigueur régissant le taux d'alcoolémie des conducteurs de véhicules automobiles. De par le caractère légal des contrôles ainsi réalisés, il est nécessaire de pouvoir étalonner les éthylomètres portables d'une manière fiable et reproductible au niveau national. Cela implique de posséder un système capable de générer des mélanges de gaz pouvant simuler la composition chimique du souffle d'un être humain pour différentes alcoolémies et pour différentes morphologies.

Si l'impact de la morphologie est assez aisément reproduit, la synthèse d'un mélange de gaz dont la composition est proche de celle du souffle d'un être humain reste difficile. Généralement, une approximation est réalisée en ne considérant que des mélanges de gaz secs. Les mélanges de gaz secs permettent de contourner les problèmes de condensation. Cette approche a été adoptée par un certain nombre de pays. Cependant le souffle d'un être humain présente un taux d'hygrométrie de 95% ; le modéliser par un mélange de gaz secs présente le risque d'obtenir des mesures fausses dans des cas où le taux d'alcoolémie est proche de la limite légale.

L'Office International de Métrologie Légale préconise dans sa recommandation R 126 éditée en 1998, l'utilisation d'un mélange de gaz ayant une humidité relative d'au moins 95% et une température de 34°C ± 0.5°C.

Il y est décrit un schéma de principe d'un banc de génération de mélange gazeux pour l'étalonnage d'éthylomètres portatifs. Notamment, le système proposé utilise un dispositif de barbotage pour humidifier un gaz porteur de façon à obtenir un mélange gazeux humide. Le barbotage consiste à faire traverser un liquide à un gaz de façon à le saturer physiquement en liquide. En pratique, le barbotage est réalisé avec un récipient clos, comprenant deux conduites, une conduite plonge dans le liquide et l'autre permet d'extraire le gaz au dessus du liquide. Un tel montage est sensible aux conditions de température et de pression régnant à l'intérieur du récipient, rendant difficile le maintien de conditions rigoureusement identiques au cours du temps.

L'objet de la présente invention est de proposer un système automatisé permettant de générer à la demande et en continu un mélange gazeux humide simulant le souffle d'un être humain et dont la composition est reproductible, connue et modifiable.

Dans un mode de réalisation, le dispositif de production d'un mélange gazeux comprend des régulateurs de débit massique gazeux pour réaliser l'admission des différents gaz entrant dans la composition du mélange, des régulateurs de débit massique liquide pour réaliser l'admission des différents liquides entrant dans la composition du mélange sans avoir besoin d'utiliser le barbotage, un moyen de contrôle de la température des gaz circulant dans le dispositif permettant d'éviter la condensation d'une partie du mélange gazeux et un moyen de commande électronique permettant de régler et de contrôler en temps réel des paramètres de fonctionnement.

Le dispositif de production comprend des régulateurs de débit massique, liquide et gazeux, qui permettent de réguler très précisément les débits de chaque constituant du mélange gazeux. Les régulateurs de débit massique liquide permettent également de vaporiser les quantités de liquide injectées qui sont ensuite emportées par les gaz porteurs. Les régulateurs de débit massique peuvent également être asservis et contrôlés par un moyen de commande en vue d'une automatisation du système de production. Enfin, vu la présence d'eau et d'alcool sous forme vaporisée, il est nécessaire de maintenir le dispositif de production à une température supérieure au point de rosée de chacun de ces constituants afin d'éviter leur condensation. La condensation aurait un double effet sur la composition du mélange gazeux par diminution de la concentration d'eau dans le mélange et par diminution de la concentration d'alcool par solubilisation dans l'eau. Il apparaît alors clairement que la maîtrise des phénomènes de condensation est très importante pour conserver une concentration appropriée du mélange.

Le dispositif comprend un régulateur de débit massique de débit gazeux d'air comprimé, un régulateur de débit massique liquide d'éthanol et un régulateur de débit massique gazeux de dioxyde de carbone connectés à un premier collecteur permettant le mélange. La sortie du premier collecteur est connectée à l'entrée d'un premier moyen d'homogénéisation. La sortie du premier moyen d'homogénéisation est connectée à un deuxième collecteur lui-même connecté à la sortie d'un régulateur de débit massique gazeux d'air comprimé et à la sortie d'un régulateur de débit massique liquide d'eau. Le deuxième collecteur est relié par une sortie à l'entrée d'un deuxième moyen d'homogénéisation. Le dispositif peut ainsi générer un mélange gazeux permettant d'étalonner un dispositif portable d'éthylométrie d'un être humain.

Le dispositif décrit précédemment peut comprendre un moyen d'analyse gazeuse connecté sur une sortie du deuxième moyen d'homogénéisation.

Le dispositif peut comprendre au moins un moyen de simulation de souffle comprenant un piston dans un cylindre comprenant au moins une entrée équipée d'une vanne commandée et au moins une sortie équipée d'une vanne commandée. Le moyen de simulation de souffle est connecté par son entrée à une sortie du deuxième moyen d'homogénéisation. Le moyen de simulation de souffle permet d'envoyer par sa sortie le mélange gazeux vers au moins un dispositif portable d'éthylométrie à étalonner.

Un moyen de maintien à température comprend une enceinte, à l'intérieur de cette enceinte, un capteur de température judicieusement positionné pour être représentatif de l'ensemble de l'enceinte, un ventilateur et une résistance de chauffage permettant de produire un flux d'air chaud afin de maintenir une température homogène et constante à l'intérieur de l'enceinte, et au moins une résistance de chauffage disposée autour des conduites de sortie du système vers les dispositifs portables d'éthylométrie à étalonner, permettant de maintenir les conduites de sortie se trouvant hors de l'enceinte à la même température que celle à l'intérieur de l'enceinte.

Le dispositif ainsi décrit peut comprendre au moins deux sorties permettant d'étalonner plusieurs dispositifs portables en parallèle, d'où un gain de temps.

Une vanne de purge permet de nettoyer le système et de réinitialiser le système avec un gaz neutre ou un gaz différent de celui utilisé avant la purge, afin d'éviter les contaminations ou les altérations de la concentration des différents composants du mélange gazeux.

Selon un autre aspect de l'invention, un procédé de production d'un flux gazeux pouvant servir à l'étalonnage de dispositifs portables d'éthylométrie d'un être humain permet d'obtenir le flux gazeux en mélangeant de l'air sec, de l'alcool et du dioxyde de carbone, puis en mélangeant le mélange gazeux obtenu avec de l'air humide, dans des quantités déterminées, afin d'obtenir un mélange gazeux pour l'étalonnage de dispositifs portables d'éthylométrie.

Une ligne de gaz permet d'injecter un mélange étalon en entrée du moyen d'analyse gazeuse.

L'ensemble des étapes est réalisé à une température comprise entre 33°C et 35°C.

L'organe de simulation de souffle injecte le mélange gazeux dans un dispositif portable d'éthylométrie à étalonner, avec des paramètres commandables.

D'autres buts, caractéristiques et avantages de l'invention apparaîtront à la lecture de la description suivante, donnée uniquement à titre d'exemple non limitatif et faite en référence aux dessins annexés sur lesquels :
- la figure 1 décrit les différents organes composant le système de production d'un flux gazeux d'après l'invention.
- la figure 2 est un organigramme des différentes étapes du procédé de production du flux gazeux.

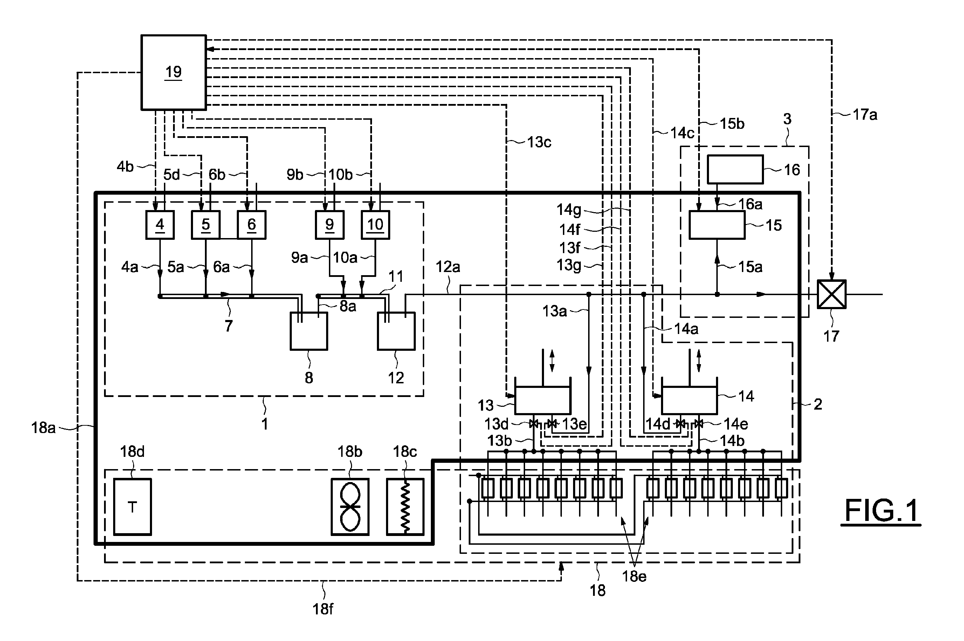

Comme illustré sur la figure 1, le système comprend un organe 1 de mélange des gaz, un organe 2 de simulation du souffle et un organe 3 d'analyse et d'étalonnage. L'organe 1 de mélange des gaz génère le mélange de gaz utilisé par l'organe 2 de simulation du souffle et pouvant être analysé par l'organe 3 d'analyse et d'étalonnage.

L'organe 1 de mélange des gaz comprend un régulateur 4 de débit massique gazeux d'air sec est capable de réguler un débit à des valeurs comprises, à titre d'exemple, entre 0 et 3 L/min. Le régulateur 4 de débit massique gazeux comprend une sortie reliée à une conduite 4a. L'organe 1 de mélange des gaz comprend également un régulateur 5 de débit massique liquide d'alcool capable de réguler un débit à des valeurs comprises, à titre d'exemple, entre 0 et 3 g/h. Le régulateur 5 de débit massique liquide comprend une sortie reliée à une conduite 5a. On entend par air sec, un air dépourvu d'humidité. L'organe 1 de mélange des gaz comprend un régulateur 6 de débit massique gazeux de dioxyde de carbone capable de réguler un débit à des valeurs comprises, à titre d'exemple, entre 0 et 3 L/min. Le régulateur 6 de débit massique gazeux comprend une sortie relié à une conduite 6a. Les conduites 4a, 5a, et 6a sont reliées à un premier collecteur 7.

Le mélange gazeux ainsi produit dans le premier collecteur 7 remplit un premier moyen 8 d'homogénéisation dont la sortie est connectée à une conduite 8a. Le premier moyen 8 d'homogénéisation peut avoir un volume compris entre 1 L et 5 L, par exemple 2 L.

Un régulateur 9 de débit massique gazeux d'air sec est capable de réguler un débit à des valeurs comprises, à titre d'exemple, entre 0 et 30 L/min. Le régulateur 9 de débit massique gazeux est relié par sa sortie à une conduite 9a. Un régulateur 10 de débit massique liquide d'eau est capable de réguler un débit à des valeurs comprises, à titre d'exemple, entre 0 et 100 g/h. Le régulateur 10 de débit massique liquide comprend une sortie reliée à une conduite 10a. Les conduites 8a, 9a et 10a sont reliées à un deuxième collecteur 11, dont la sortie est reliée à l'entrée du deuxième moyen 12 d'homogénéisation assurant l'homogénéisation du mélange des gaz cités. Le deuxième moyen 12 d'homogénéisation peut avoir un volume compris entre 1 et 10 L, de préférence 7 L. La sortie du deuxième moyen 12 d'homogénéisation est reliée à une conduite 12a qui permet d'acheminer le mélange gazeux vers les organes 2 et 3.

L'organe 2 de simulation du souffle comprend ici deux moyens de simulation du souffle humain. L'organe 2 de simulation du souffle reçoit le mélange gazeux par la conduite 12a.

Une première conduite 13a est piquée sur la conduite 12a et amène une partie du mélange gazeux à un premier moyen 13 de simulation du souffle humain. Le moyen 13 de simulation du souffle humain comprend un cylindre présentant une entrée munie d'une vanne 13d, une sortie munie d'une vanne 13e, et un piston permettant d'expulser le gaz contenu dans le cylindre vers la conduite 13b reliée à la sortie du cylindre. La conduite 13b se sépare en plusieurs sorties en parallèle pouvant être reliées chacune à un éthylomètre.

Une deuxième conduite 14a est piquée sur la conduite 12a et amène une partie du mélange gazeux à un deuxième moyen 14 de simulation du souffle humain. Le moyen 14 de simulation du souffle humain comprend un cylindre présentant une entrée munie d'une vanne 14d, une sortie munie d'une vanne 14e, et un piston permettant d'expulser le gaz contenu dans le cylindre vers la conduite 14b reliée à la sortie du cylindre. La conduite 14b se sépare en plusieurs sorties en parallèle pouvant être reliées chacune à un éthylomètre.

Chacun des deux moyens de simulation du souffle humain reçoit sur son entrée le mélange de gaz, qui est aspiré par retrait du piston dans le cylindre alors que la vanne d'entrée est ouverte et la vanne de sortie fermée. Une fois que le volume adéquat a pénétré dans le cylindre, la vanne d'entrée est fermée et la vanne de sortie ouverte, le piston est enfoncé pour éjecter les gaz dans la sortie du cylindre avec une vitesse contrôlée de façon à moduler la durée, la vitesse d'éjection du mélange gazeux dans un ou plusieurs éthylomètres. Par éthylomètre, on entend un éthylomètre nécessitant ou non un rinçage entre chaque mesure par de l'air ambiant transitant par le tube d'entrée. Il est à noter qu'une des sorties du système de génération de mélange gazeux permet de raccorder un éthylomètre à fonctionnement manuel. Le contrôle de ces paramètres permet de simuler différents souffles issus de différentes morphologies humaines.

La conduite 12a se poursuit jusqu'à l'organe 3 d'analyse et d'étalonnage. Cet organe comprend un analyseur 15 du mélange gazeux, relié à la conduite 12a par une conduite 15a. L'analyseur 15 du mélange gazeux peut comprendre un analyseur à détecteur d'ionisation de flamme. Cet analyseur est capable de détecter la teneur en alcool dans une gamme de concentrations comprises entre 55 et 1660 ppm. Une conduite 16a relie le moyen d'analyse à une source 16 de gaz d'étalonnage, par exemple une bouteille, permettant d'étalonner l'analyseur 15 du mélange gazeux. La conduite 12a se termine par une vanne 17 de fuite, permettant de purger les organes 1 et 2 ainsi que la conduite 15a. Purger le système permet d'éliminer toute trace des gaz employés et d'éviter une contamination et une altération des concentrations du mélange gazeux d'un étalonnage à un autre. Cette purge peut être réalisée en utilisant l'air sec comme gaz neutre, bien que tout autre gaz non réactif tel que de l'hélium ou de l'azote peut être utilisé. En fonctionnement normal, la vanne 17 de fuite est ouverte et crée une fuite continue, permettant de conserver un débit continu des régulateurs de débit massique et une pression constante dans les collecteurs.

Un moyen 18 de contrôle de la température des gaz circulant dans le dispositif permet de contrôler la température du système. Les différents organes du système de production sont regroupés à l'intérieur d'une enceinte 18a isolée thermiquement de l'extérieur dont la température est maintenue à une température comprise entre 33°C et 35°C, de préférence 34°C. Le moyen 18 de contrôle de la température des gaz circulant dans le dispositif comprend un ventilateur 18b, une résistance 18c de chauffage de l'air soufflé, un capteur 18d de température représentatif de la température à l'intérieur de l'enceinte. Le moyen 18 de contrôle de la température des gaz circulant dans le dispositif peut comprend également des résistances chauffantes 18e autour des sorties vers les dispositifs portables d'éthylométrie.

Un moyen 19 de commande électronique contrôle les régulateurs de débit massique 4, 5, 6, 9 et 10 par les liaisons 4b, 5d, 6b, 9b et 10b. Le moyen 19 de commande électronique commande également les organes 13 et 14 de simulation du souffle humain par les liaisons 13c et 14c, les vannes 13d, 13e, 14d et 14e par les connexions 13f, 13g, 14f et 14g respectivement. Le moyen 19 de commande électronique commande également la vanne 17 de fuite par la liaison 17a et le moyen 13 d'analyse par la liaison bidirectionnelle 13b. Enfin, le moyen 19 de commande électronique commande également le moyen 18 de contrôle de la température des gaz circulant dans le dispositif par une connexion 18f.

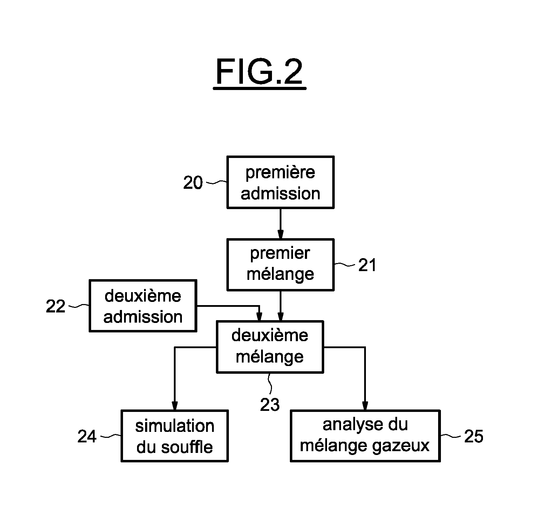

La figure 2 est un organigramme des différentes étapes du procédé de production du flux gazeux. Le procédé débute par une première admission des gaz à l'étape 20. Les régulateurs de débit massique 4, 5 et 6 débitent, et l'air sec, l'alcool et le dioxyde de carbone admis dans le circuit. On poursuit à l'étape 21 par le mélange des ces gaz dans le premier collecteur 7 et le premier moyen 8 d'homogénéisation. A l'étape 22, les régulateurs de débit massique 9 et 10 débitent, et l'air humide est admis dans le circuit. On poursuit à l'étape 23 par le mélange de l'air humide avec le mélange de gaz issu de l'étape 21 dans le premier collecteur 7 et le premier moyen 8 d'homogénéisation. Le mélange gazeux obtenu est ensuite envoyé vers l'organe 2 de simulation du souffle à l'étape 24. Le mélange gazeux est aspiré dans le cylindre d'un moyen 13 de simulation de souffle alors que la vanne 13d d'entrée du moyen de simulation de souffle est ouverte et la vanne de sortie 13e fermée. Les vannes voient leurs positions inversées, la vanne d'entrée 13d fermée et la vanne de sortie 13e ouverte, le piston est alors enfoncé afin d'éjecter le gaz par la sortie du moyen 13 de simulation de souffle vers les dispositifs d'éthylométrie portables à étalonner. L'étape 25 est une alternative au cours de laquelle l'envoi du mélange gazeux vers l'analyseur 15 de mélange gazeux permet la confrontation avec des mesures préalablement réalisées sur un gaz étalon.

En d'autres termes, le moyen de commande déclenche l'admission de l'air, de l'alcool, de l'eau et du dioxyde de carbone dans des proportions prédéterminées. Air, alcool et dioxyde de carbone sont mélangés et homogénéisés. Le premier mélange est mélangé de nouveau avec de l'air humide issu du mélange d'eau et d'air et homogénéisé. Le mélange gazeux obtenu est envoyé vers l'analyseur ou vers les moyens de simulation de souffle suivant la phase de fonctionnement du système de production. Dans le cas d'un envoi vers les moyens de simulation de souffle, le mélange est admis dans un cylindre puis expulsé par un piston vers la conduite de sortie. Cette expulsion peut voir certains de ses paramètres modulés, comme par exemple et de façon non limitative, la durée, l'intensité et le volume expulsé, de façon à simuler les caractéristiques du souffle d'un être humain pouvant présenter différentes morphologies.

Le système de génération de mélange gazeux permettant de respecter les recommandations de l'OIML, notamment concernant le taux d'humidité tout en proposant des capacités de production en continu et de façon automatisée. Ce système est adaptable à d'autres sources de gaz pour prendre en compte l'effet sur les éthylomètres d'autres gaz pouvant être générés par un être humain. Une analyse en ligne est possible afin de contrôler le mélange gazeux généré. Enfin, ce système permet d'ajuster les paramètres de souffle pour simuler l'influence de différentes morphologies.

## Revendications

1. Dispositif de production d'un mélange gazeux **caractérisé par le fait qu'**il comprend des régulateurs de débit massique gazeux pour réaliser l'admission de gaz entrant dans la composition du mélange, des régulateurs de débit massique liquide pour réaliser l'admission de liquides entrant dans la composition du mélange, un moyen (18) de contrôle de la température des gaz circulant dans le dispositif permettant d'éviter la condensation d'une partie du mélange gazeux et un moyen (19) de commande électronique permettant de régler et de contrôler en temps réel des paramètres de fonctionnement.

2. Dispositif selon la revendication 1, dans lequel un régulateur (4) de débit massique gazeux d'air comprimé, un régulateur (5) de débit massique liquide d'éthanol et un régulateur (6) de débit massique gazeux de dioxyde de carbone sont connectés à un premier collecteur (7) permettant le mélange et la sortie du premier collecteur (7) est connectée à l'entrée d'un premier moyen (8) d'homogénéisation, la sortie du premier moyen (8) d'homogénéisation étant connectée à un deuxième collecteur (11) lui-même connecté à la sortie d'un régulateur (9) de débit massique gazeux d'air comprimé et à la sortie d'un régulateur (10) de débit massique liquide d'eau, le deuxième collecteur (11) étant relié par une sortie à l'entrée d'un deuxième moyen (12) d'homogénéisation, pour générer un mélange gazeux permettant d'étalonner un dispositif portable d'éthylométrie d'un être humain.

3. Dispositif selon la revendication 2 comprenant un moyen (15) d'analyse gazeuse connecté sur une sortie du deuxième moyen (12) d'homogénéisation.

4. Dispositif selon l'une des revendications 2 ou 3, comprenant au moins un moyen (13) de simulation de souffle comprenant un piston dans un cylindre comprenant au moins une entrée équipée d'une vanne (13d) commandée et au moins une sortie équipée d'une vanne (13e) commandée, connecté par son entrée à une sortie du deuxième moyen (12) d'homogénéisation et permettant d'envoyer par sa sortie le mélange gazeux vers au moins un dispositif portable d'éthylométrie à étalonner.

5. Dispositif selon l'une des revendications précédentes, comprenant un moyen (18) de contrôle de la température des gaz circulant dans le dispositif, comprenant une enceinte (18a), un ventilateur (18b), une résistance de chauffage (18c), un capteur (18d) de température placé de façon à mesurer une température représentative de la température à l'intérieur de l'enceinte et au moins une résistance (18e) de chauffage disposée autour de conduites de sortie vers des dispositifs portables d'éthylométrie à étalonner.

6. Dispositif selon l'une des revendications précédentes, comprenant au moins deux sorties permettant d'étalonner plusieurs dispositifs portables en parallèle.

7. Dispositif selon l'une des revendications précédentes comprenant une vanne (17) permettant de purger les collecteurs.

8. Procédé de production d'un flux gazeux pouvant servir à l'étalonnage de dispositifs portables d'éthylométrie d'un être humain **caractérisé par le fait que** l'on mélange de l'air sec, de l'alcool et du dioxyde de carbone, puis que l'on mélange le mélange gazeux obtenu avec de l'air humide, dans des quantités déterminées, afin d'obtenir un mélange gazeux pour l'étalonnage de dispositifs portables d'éthylométrie.

9. Procédé selon la revendication 8 dans lequel une ligne de gaz injecte un mélange étalon en entrée d'un moyen (15) d'analyse gazeuse.

10. Procédé selon l'une des revendications 8 ou 9 dans lequel les étapes sont réalisées à une température comprise entre 33°C et 35°C.

11. Procédé selon l'une des revendications 8 à 10, dans lequel l'organe de simulation de souffle injecte le mélange gazeux dans un dispositif portable d'éthylométrie à étalonner.
